# EUROPEAN PATENT APPLICATION

(11) **EP 4 141 110 A1**
(43) Date of publication of application: **01.03.2023**
(21) Application number: 20932307.0
(22) Date of filing: 24.04.2020
(51) Int. Cl.: C12N 7/01, C12N 7/04, C12N 15/63

(54) **METHOD FOR PRODUCING PARTICLES OF BACTERIOPHAGES OF THE GENUS LEVIVIRUS**

(71) Applicant: OBSHCHESTVO S OGRANICHENNOY OTVETSTVENNOST'YU "INGENIK", Moscow, 119192 (RU)
(72) Inventor: SIVOV, Igor Gennadievich, Moscow, 129626 (RU); FIRSOV, Igor Sergeevich, Moscow, 121357 (RU)
(74) Representative: Ilievski, Bogoljub
(86) International application number: PCT/RU2020/000198
(87) International publication number: WO 2021/215952

(57) **Abstract**

This group of inventions relates to medicine and biology and concerns a technologically simple method to produce the particles of the bacteriophages of the Levivirus family (MS2-like), devoid of LPS toxicity, and a method of their biomineralization, which provides the prerequisites for the study of their use for a targeted delivery to the malignant tumor cells (including the endothelial cells of the pathological circulatory network) enriched in integrin ανβ3, which allows to drastically reduce their overall toxicity.

## Description

### FIELD OF THE INVENTION

The group of inventions relates to medicine and biology and concerns a technologically simple method for producing the particles of the bacteriophages of the Levivirus family (MS2-like), devoid of LPS toxicity, and a method of their biomineralization, which provides the prerequisites for the study of their use for a targeted delivery to the malignant tumor cells (including the endothelial cells of the pathological circulatory network) enriched in integrin αᵥβ₃, which allows to drastically reduce their overall toxicity.

### STATE OF THE ART

Phage-like particles (VLPs) are obtained for immunological purposes [US Patent Application 201700008778; US Patent Application 20190315807], as well as for a clinical usage as targeted medication delivery systems [US Patent 8324149]. Guided by the above tasks, the methods for obtaining VLPs in large quantities have been developed, which are based on genetic engineering methods [US Patent 5534257; WO9306921; US Patent Application 20190307819], while using various cell cultures (bacteria, microscopic fungi, insect or animal cells, as well as human cell lines).

Virus-like particles (VLPs) are multimeric nanostructures assembled from native viral structural proteins, but devoid of any genetic material. The VLPs consist of high-density displays of viral surface proteins and as such are a highly adaptive tool for various applications (e.g., antibody development, a delivery system, a membrane protein expression). The VLPs can also provide tissue-specific targeting, as they originate from their own virus. On the other hand, the surface of the particles is susceptible to a reaction of chemical conjugation, which makes it possible to create a tissue-specific ligand on its surface. In this case, the VLP particle becomes, as the developers put it, a display that exhibits its ability to find cellular "targets."

In the early work with bacteriophages [Zeltins A. Construction and characterization of virus-like particles: a review//Mol Biotechnol. 2013; v.53(No 1), pp.92-107], genetic engineering methods for creating the VLPs of Lentivirus were described, which were obtained by sequential operations: cloning the cDNA of the genomic RNA sequences of these bacteriophages, their expression in bacterial E.coli cells, and then the release of the particles using detoxification methods.

Further development of the method of obtaining the VLPs of Lentivirus was aimed at the use of various expression systems of genetic structures [Blazeck J., Alper H.S. Promoter engineering: recent advances in controlling transcription at the most fundamental level//Biotechnol J. 2013; v.8(No 1),pp.46-58], in which new genetic engineering "novelties" were introduced in the form of cloning the tandems of the gene of the shell comprising, in order to obtain the desired immunological response, the additional DNA-coding fragments [Zhai L., J. Peabody, Y.-Y. S. Pang, J. Schiller, B. Chackerian, E. Tumban. A Novel Candidate MS2 Phage VLP Vaccine Displaying a tandem HPV L2 Peptide offers Similar Protection in Mice to Gardasil-9// Antiviral Res. 2017 Nov; 147: 116-123].

Several patents relating to the above-disclosed methods for obtaining the VLPs of Lentivirus, including US Patents 4722840; 5534257; 7494656; 8324149, as well as US Patent Applications 20130224828; 20140302593; 20160090403; 20160208221, and the contents of which are systematized in publications [P. Pumpens et al. The True Story and Advantages of RNA Phage Capsids as Nanotools//Intervirology 2016;v.59(2), pp.74-110; Rohovie M.J., Nagasawa M., Swartz1 J.R. Virus-like particles: Next-generation nanoparticles for targeted therapeutic delivery// Bioengineering & Translational Medicine 2017; v.2(1), pp. 43-57], have, as a common disadvantage, a significant list of methods of obtaining that require highly qualified workers and energy costs. In addition, the use as host cells of the bacteria with an LPS antigen toxic to humans and animals makes it necessary to use expensive sorbents at an isolation stage.

The chemical modification of the surface of the VLPs of Lentivirus, necessary to give a "target" ability and to obtain displays from them, was developed as a conjugation method and as a method for obtaining capsid proteins with additional peptide sequences, similar to a phage display method [Smith G.P. Filamentous fusion phage: novel expression vectors that display cloned antigens on the virion surface//Science 1985; v.228(No 4705), pp.1315-1317], mentioned above. The administration into the structure of the capsid B protein was carried out taking into account the data [van Meerten D., R. C. L. Olsthoorn, J. van Duin, R. M. D. Verhaert. Peptide display on live MS2 phage: restrictions at the RNA genome level//Journal of General Virology 2001, v.82(No 7), pp.1797-1805].

Among all the variety of chemical, genetic and physical and chemical methods, a simple reaction of conjugation of penicillins with proteins attracts attention. Penicillins are a large family of compounds, which common structural basis is a β-lactam ring. The β-lactam ring is a target for nucleophilic attack of free amino groups of the proteins, leading to opening the ring and covalent amide binding of the penicilloyl group. The penicilloyl configuration, in which the hapten determinant is covalently bound to the *ε*-amino groups of lysine residues of the proteins, accounts for more than 90% of the products of the conjugation reaction with the proteins. Other conjugation products can be formed by degradation of penicillin in a solution to penicillen or penicilloyn acid, which form disulfide bonds with sulfhydryl groups of cysteine, producing penicillen- and penicillamine-protein conjugates, respectively [Weltzien H.U., E. Padovan. Molecular Features of Penicillin Allergy//Journal of investigative dermatology 1998, v.110(3), pp. 203-206].

The only addition to obtaining these conjugates is the preliminary synthesis of the peptide derivatives of 6-amino penicillin acid, obtaining of which is disclosed in patents [US Patents 3957764; 4229348; 4810777; DE 69731747].

The filling of the obtained VLPs phages of the Lentivirus family with organometallic compounds (a pharmaceutical medication having an activity center containing a metal atom) is provided by genomic RNA or its fragments [Liu L., Pu X., Yin G., Chen X., Yin J., Wu Y. Biomimetic Mineralization of Magnetic Iron Oxide Nanoparticles Mediated by Bi-Functional Copolypeptides//Molecules 2019; v.24(No 7), pp. 1-16], as well as the "internal" ligands introduced into the structure of the capsid of VLPs of Lentivirus after obtaining the latter. Currently, 89 metal medications are used in medicine [Barry N.P., Sadler P.J. Exploration of the medical periodic table: towards new targets//Chem Commun (Camb). 2013, v.49 (No 45), pp.5106-5131], which, often, have a low "therapeutic ratio" or "therapeutic window" (the ratio of two doses of the medication, where the first causes side effects in the disease, and the second causes the severity of the administration, unrelated to the target indication). For example, the ratio of the toxic dose in 50% of cases, TD₅₀ to the dose at which the medication exhibits the desired pharmacological effect, the effective dose in 50% of cases, ED₅₀). It is obvious that the targeted delivery "expands the therapeutic window" by creating a high local concentration of the medication. Previously, the fundamental possibility of using the mentioned technology was established, applicable to the MS2 phage filled with monovalent thallium salts [Firsov I.S., Sivov I.G. Remission in the Patient with Malignant Pleural Mesothelioma: A Case Report// J Clin Case Rep 2019, v. 9(No 2), pp. 1214-1217]. The level of toxicity of the medications prepared on the basis of the monovalent thallium salts in this case was reduced by 10,000 times.

### DISCLOSURE OF THE GROUP OF INVENTIONS

The technical result of the claimed group of inventions is in the development of the technologically simple method for creating the particles of the bacteriophages of the Levivirus family (MS2-like), devoid of LPS toxicity, and the method of their biomineralization, which provides the prerequisites for the study of their use for the targeted delivery to the malignant tumor cells (including the endothelial cells of the pathological circulatory network) enriched in integrin αᵥβ₃. which allows to drastically reduce their overall toxicity.

The technical result is achieved by the method for obtaining the particles of the bacteriophages of the Levivirus family, which includes the following steps of:
(a) obtaining a line of the pseudolysogenic host cells multiplying at a temperature of ≤ 23° C, devoid of toxic lipopolysaccharide and comprising:
   - a sequence of the nucleic acid encoding T7 phasmid with a fragment of the genomes of the bacteriophages of the Levivirus family;
   - a sequence of the nucleic acid of T7 phasmid encoding the proteins of the capsid of the particles of the bacteriophages of the Levivirus family;
   - a sequence of the nucleic acid of T7 phasmid encoding the proteins of the DNA- and RNA-polymerase of T7 phage and devoid of the morphogenesis genes;
(b) initiating overexpression of the genes encoding the proteins of the capsid of the particles of the bacteriophages of the Levivirus family at an increase in temperature ≥ 28° C;
(c) obtaining the particles of the bacteriophages of the Levivirus family with simultaneous destruction of the host cells, wherein these bacteriophage particles have morphological similarity with the natural phage particles.

In addition, the technical result, namely, to significantly reduce the overall toxicity of the particles loaded with metal medications, is achieved by the method of biomineralization of the particles of the bacteriophages of the Levivirus family, which includes the following steps of:
(a) complete removal of RNA from the particles of the bacteriophages of the Levivirus family;
(b) obtaining the complex of the metal medications with hairpin RNA (shRNA) binding at least 200 cations;
(c) "loading" the complex of the metal medications with hairpin RNA (shRNA) binding at least 200 cations, into the particles of the bacteriophages of the Levivirus family, devoid of RNA, "loosened" by trimethylamine N-oxide;
(d) the non-enzymatic addition of the ligand to the biomineralized particles suitable for creating a targeted delivery system to the malignant tumor cells, including to the cells enriched in integrin αᵥβ₃;
(e) determination of the delivery specificity and sensitivity using a mixture model of a PC3 cell line, one of which is enriched in integrin αᵥβ₃.

Further, the claimed group of inventions will be described in more detail only as an illustration of the following examples. It is assumed that the examples serve to illustrate the group of inventions and should not be considered as limiting the universality of the disclosure of the description in this source [US Patent Application 20180250331].

In the production of the phages of the genus Lentivirus, affecting "male" bacterial strains, the main difficulty is to preserve the sensitivity that bacterial cells, in the presence of the bacteriophage, quickly lose [Spengler G, Molnár A, Schelz Z, Amaral L, Sharples D, Molnár J. The mechanism of plasmid curing in bacteria//Curr Drug Targets. 2006; v.7(No 7); pp.823-41; Getino M., D.J. Sanabria-Rios, R. Fernández-López, J. Campos-Gómez, J.M. Sánchez-López, A. Fernández, N.M. Carballeira, F. de la Cruz. Synthetic Fatty Acids Prevent Plasmid-Mediated Horizontal Gene Transfer//mBio. 2015; v.6(No 5), pp. e01032-15]. At a genetic level, this phenomenon is expressed in the elimination of the "male" plasmids from the population, and at a biotechnological level it leads to an unstable "harvest" of the phage. This explains the difference in the selling price of the therapeutic phages (about 10 roubles/ml) and one of the phages of the genus Lentivirus - MS2 (14,000 roubles/ml). It should be pointed out that the number of the infectious particles in the first case exceeds the same indicator in the case of MS2 by 1,000 times.

The authors of the group of inventions see a reduction in the cost of medications of the phages of the genus Lentivirus in the application of the phenomenon of pseudolysogeny [Bolshakova T.N., Dobrynina O.Yu. Karataev G.I., Sivov I.G. Pseudolysogeny in T7 Phage and Its Use (Within the Framework of the Scientific and Practical Conference with the International Participation "Bacteriophages: Theoretical and Practical Aspects of Application in Medicine, Veterinary Medicine and Food Industry")//Moscow, 2016, Russia]. The genome of defective T7 phages capable of causing pseudolysogeny comprises a strictly polar mutation. The strictly polar mutation is mapped behind the A3 promoter, which leads to a significant decrease in the frequency of transcription of the genome of the phage (< 10²). When bacteria of pseudolysogenic clones are infected with T7 helper phage in the offspring of the phages, the ratio of the phage particles and the particles capable of causing pseudolysogeny was 1:1 (Hft lysates). The choice of T7 phage as a model of pseudolysogeny is based on its ability to cause infection of a wide range of host bacteria (*Escherichia coli*, *Salmonella enterica Typhimurium*, *Shigella flexnery*, *Bordetella parapertussis, Pseudomonas aeruginosa, Pseudomonas putida, Erwinia carotovorum*, *Yersinia enterocolitica*, *Francisella spp*, *Brucella abortus*), a number of which comprises a non-toxic to humans LPS-antigen.

To increase efficiency and reduce the overall toxicity, chemotherapy medications are encapsulated, for example, in the phages' virions of the genus Lentivirus [US Patent 5677124, 6159728, US Patent Application 20100167981; Carlee E. Ashley et al. Cell-Specific Delivery of Diverse Cargos by Bacteriophage MS2 Virus-Like Particles//ACS Nano. 2011; 5(7): 5729-5745].

In the full-fledged virions of the phages of the genus Lentivirus, the capsids comprise cone-shaped pores: an inlet diameter is 5-7 nm and an internal diameter is 1.5 - 2 nm [Michel T. Dedeo, Daniel T. Finley, Matthew B. Francis. Chapter 8 - Viral Capsids as Self-Assembling Templates for New Materials//page 366. In book "Molecular Assembly in Natural and Engineered Systems" (Edited by S. Howorka) 2011, v. 103, pp. 1-401]. The success of mineralization depends on the preservation of the crystallization property [Aljabali A. A., Sachin N. Shah, Richard Evans-Gowing, George P. Lomonossoff, David J. Evans. Chemically-coupled-peptide -promoted virus nanoparticle templated mineralization// Integr. Biol., 2011, 3, 119-125], when the virions are purified from genomic ssRNA either completely or partially [Hooker J. M., E. W. Kovacs, M. B. Francis. Interior Surface Modification of Bacteriophage MS2//J. AM. CHEM. SOC. 2004, v. 9(No 12), pp. 3718-3719]. In the case of obtaining the particles with the shortened RNA genome capable of diverse packaging [Sun L.-Z., D. Zhang, S.-J. Chen. Theory and Modeling of RNA Structure and Interactions with Metal Ions and Small Molecules//Annu Rev Biophys. 2017; v.46 (No 1), pp. 227-246], it is possible, based on the appropriate calculations, to predict an increase in the number of duplex loops binding the heavy metal cations [Bonilla S., C. Limouse, N. Bisaria, M. Gebala, H. Mabuchi, D. Herschlag. Single-Molecule Fluorescence Reveals Commonalities and Distinctions among Natural and in Vitro-Selected RNA Tertiary Motifs in a Multistep Folding Pathway// J. Am. Chem. Soc. 2017, v.139 (No 51), pp.18576-18589].

In the case of a complete hydrolysis of the genomic ssRNA and obtaining the VLPs, the genetically modified internal surface of the virion is subjected to a chemical modification that allows the attachment of the metal medications [US Patent 8187607; US Patent Application 20110286971].

A representative of the phages of the genus Lentivirus, the MS2 bacteriophage loaded with the heavy metal salts, has a basis for the safe use in the medical practice, as shown by the practical application of iRGD-MS2 display [US Patent Application 20140106982; Ashley C. E. , Eric C. Carnes, Genevieve K. Phillips, Paul N. Durfee, Mekensey D. Buley, Christopher A. Lino, David P. Padilla, Brandy Phillips, Mark B. Carter, Cheryl. L. Willman, C. Jeffrey Brinker, Jerri do Carmo Caldeira, Bryce Chackerian, Walker Wharton, David S. Peabody. Cell-Specific Delivery of Diverse Cargos by Bacteriophage MS2 Virus-Like Particles// ACS Nano. 2011 Jul 26; 5(7): 5729-5745; Diaz D. , Andrew Care, Anwar Sunna. Bioengineering Strategies for Protein-Based Nanoparticles//Genes (Basel). 2018; v.9 (No 7), pp. 370-400], including in the case of filling the particles with monovalent thallium nitrate [RF Patent 2599462, Kolesanova E. F., Melnikova M.V., Bolshakova T. N., Rybalkina E. Yu., Sivov I.G. Bacteriophage MS2 is a delivery vehicle for targeted chemotherapy of solid tumors //Acta Naturae. 2019. V. 11. No 2 (41). Pp. 98 -101; Firsov I.S., Sivov I.G. Remission in the Patient with Malignant Pleural Mesothelioma: A Case Report// J Clin Case Rep 2019, v. 9(No 2), pp. 1214-1217]. The concentration of Tl⁺¹ in the dose of the medication mentioned in the RF patent is 5 times less than DLM for the salts of the mentioned ion. Using an example of the targeted delivery of the monovalent thallium ion, the possibility of reducing toxicity to the levels of the values below those that are acceptable was already shown [TOXICOLOGICAL REVIEW OF THALLIUM AND COMPOUNDS // U.S. Environmental Protection Agency, 2009].

In the this group of the authors of the inventions, cisplatin (cis-dichlorodiamminplatin) (II) or ammonium hexachloroplatinate (IV); ruthenium nitrosonitroamine, aurothiosulfate, iron polymaltosate hydroxide, silver, copper or zinc nitrates, as well as gallium trinitrate, vanadium salts and thallium salts of various valence were administered into the composition of the VLPs of the phages of the Lentivirus family.

In one aspect of this group of inventions, by an analogy with the disclosed method in [US Patent Applications 20090092538; 20180103645; 20170071211], filling was carried out:
- in the case of cisplatin, by the methods disclosed in [US Patent 7594884; US Patent Application 20190350871], with the modifications, but in relation to the structures of the VLPs of the phages of the Lentivirus family;
- in the case of ammonium hexachloroplatinate (IV), by the methods disclosed in [US Patent 7569606; EP 2958553] with the modifications, but in relation to the structures of the VLPs of the phages of the Lentivirus family;
- in the case of ruthenium nitrosonitroamine, aurothiosulfate, iron polymaltosate hydroxide, silver, copper or zinc nitrates, gallium trinitrate, thallium salts and vanadium salts, by the method disclosed in [US Patent 7594884; US Patent Application 20190350871] with the modifications, but in relation to the structures of the VLPs of the phages of the Lentivirus family;

In another aspect of this group of inventions, the "empty" virions were obtained by the temporary temperature destruction and assembly in the presence of trimethylamine N-oxide (TMAO), in the same way [US Patent Application 20040152181; WO1995031476; RF Patent 2599462].

In accordance with another aspect of the group of inventions, the phage particles of the genus Lentivirus may comprise an "outer" ligand that ensures its interaction with the receptor present on the target cell and is capable of forming a "display" due to the non-enzymatic reaction of the interaction of the 6-peptidylopenicillic acid radical with the surface lysine or cysteine amino acid radicals [N.F. Adkinson, Jr; L. M. Mendelson; C. Ressler; J. C. Keogh. Penicillin minor determinants//Ann Allergy Asthma Immunol. 2018; v.121; pp.537-544]. With the administration of such a phage display medication obtained in the reactions with lysyl or cysteyl, an immunological reaction can be avoided, as disclosed in the description to the application [WO 2020030954].

The toxicity of the metal medications having a platinum atom in the structure is associated with neurotoxicity, which is induced by the formation of the adducts with the DNA [O. Kanat, Hulya Ertas, Burcu Caner. Platinum-induced neurotoxicity: A review of possible mechanisms// World J Clin Oncol 2017; v.8(4), pp. 329-335]. In accordance with the possible side effects of the platinum medications, the minimum therapeutic doses are within the values of 10-100 µg/m² [US Patent Application 20180078551]. The toxicity of the metal medications having a gold atom in the structure is described, where its tolerability is indicated within the values of 1-500 mg/kg [US Patent Application 20180303954]. The gold compounds (III) according to the invention [US Patent Application 20150182489] have an antitumor activity. Other metal medications having an antitumor potential with an acceptable level of toxicity are mentioned in US Patent 10098952.

Previously, a method of the targeted delivery of the salts to the solid tumor cells [RF Patent 2599462; the method for obtaining such particles is mineralization] of the monovalent thallium ion having the highest toxic potential was offered [Toxicological review of thallium and compounds//U.S. Environmental Protection Agency, Washington, DC. September 2009]. In addition, the diagnostic methods were offered based on the targeted delivery to the solid tumor cells **^{99m}** Tc [US Patent Application 20150125533] or the oral delivery of the therapeutic and detectable agents to a diseased tissue [US Patent Application 20180000949], as well as to the vascular network of the tumor [US Patent Application 20190022170].

However, the issue of the targeted delivery of the metal medications and reducing their toxic effects on the recipient's body remains unresolved [Topcul M, Cetin I. Endpoint of cancer treatment: targeted therapies//Asian Pac J Cancer Prev. 2014; v.15(11); pp. 4395-403; Maeda H, Khatami M. Analyses of repeated failures in cancer therapy for solid tumors: poor tumor-selective drug delivery, low therapeutic efficacy and unsustainable costs//Clin Transl Med. 2018; v.7(1); pp.11 - 30].

Thus, from the state of the art, the mineralization methods are known for obtaining the nanoparticles for the medical purposes in apoferritin [US Patent 9,925,592], as well as in tobacco mosaic virus (TMV) [US Patent Application 20180256758].

Previously, the fundamental possibility of using the mentioned technology was established, applicable to the MS2 phage filled with the monovalent thallium salts [Firsov I.S., Sivov I.G. Remission in the Patient with Malignant Pleural Mesothelioma: A Case Report// J Clin Case Rep 2019, v. 9(No 2), pp. 1214-1217]. In the described case, the level of toxicity of the medications prepared on the basis of the monovalent thallium salts was reduced by more than 10,000 times, which makes it possible to expect a similar result with Cr(VI), for which DCL = 50-150 mg/kg. The experts of the World Health Organization (WHO) recommend a maximum permissible concentration of 50 µg per aliter of chromium (VI) in drinking water. The metal medications were administered into the composition of VLPs, "empty" virions and other methods of "loading" with medications, as the variants of the known mineralization methods [US Patent Application 20140377289].

In one aspect of the group of inventions, the VLPs were obtained using the pseudolysogeny system of T7 bacteriophage [Bolshakova T.N., Dobrynina O.Yu. Karataev G.I., Sivov I.G. Pseudolysogeny in T7 Phage and Its Use (Within the Framework of the Scientific and Practical Conference with the International Participation "Bacteriophages: Theoretical and Practical Aspects of Application in Medicine, Veterinary Medicine and Food Industry")//Moscow, 2016, Russia].

In another aspect of the group of inventions, the "empty" virions with the necessary point substitutions of the amino acids of the capsid protein in the phages of the genus Levivirus were obtained by direct hydrolysis of the genomic ssRNA from the "normal" virions, in the same way [EP 1736538];

In one variant, the metal-binding agent was obtained in the same way [US Patent Application 20140377289]. In accordance with this aspect of this group of inventions, the peptides of the general structure (HHQG)**ₙ** are offered, which are embedded in the sequences encoding the surface of B protein, which faces the internal part of the capsid.

In the context of this group of inventions, the term "administration" and the variants of this term, including "administration", include contacting. For any particular case, the appropriate "effective quantity" can be determined by person skilled in the art using only routine experiments.

The above and other tasks, peculiarities, advantages, as well as the technical significance of this group of inventions will be more clear from the following detailed description with the references to the accompanying figures.

### BRIEF DESCRIPTION OF THE FIGURES

Figure 1 shows the structure of T7 cointegrates::pMS3,6 VRep (or isolates fr; M12; DL1; DL16; J20; ST4; R17 - SEQ ID NO 1) or T7::pGA2,6 VRep (or isolates KU1; T72; DL10; DL20- SEQ ID NO 2).
Figure 2 shows obtaining and analysis of the particles of the phages of the genus Lentivirus (the typical results are given).
Figure 3 shows the determination of the structure of the obtained VLPs.
Figure 4 shows the change in the size of the obtained VLPs after filling them with AgNOs (the concentration is 25 mM).
Figure 5 shows AFM (atomic force microscopy) of MS2 bacteriophage and the obtained phages of the genus Levivirus treated with anti-poly-lysin IgG1 antibodies.
Figure 6 shows the modification of the particles of the phage of the genus Levivirus by the peptide (HHQG)**₄** (SEQ ID NO 8), which causes a change in the size of the particles with a poly-lysin insert.
Figure 7 shows the non-enzymatic conjugation of peptidyl-6APA with B protein of the Levivirus particles.
Figure 8 shows an isotopic analysis of the detection of a stable isotope.
Figure 9 shows the results of determining the accuracy of the delivery by the method of flow fluorimetry from the mixture of the cells enriched in ανβ3 integrin and devoid of it (lines PC3 and HEK293).

### IMPLEMENTATION OF THE GROUP OF INVENTIONS

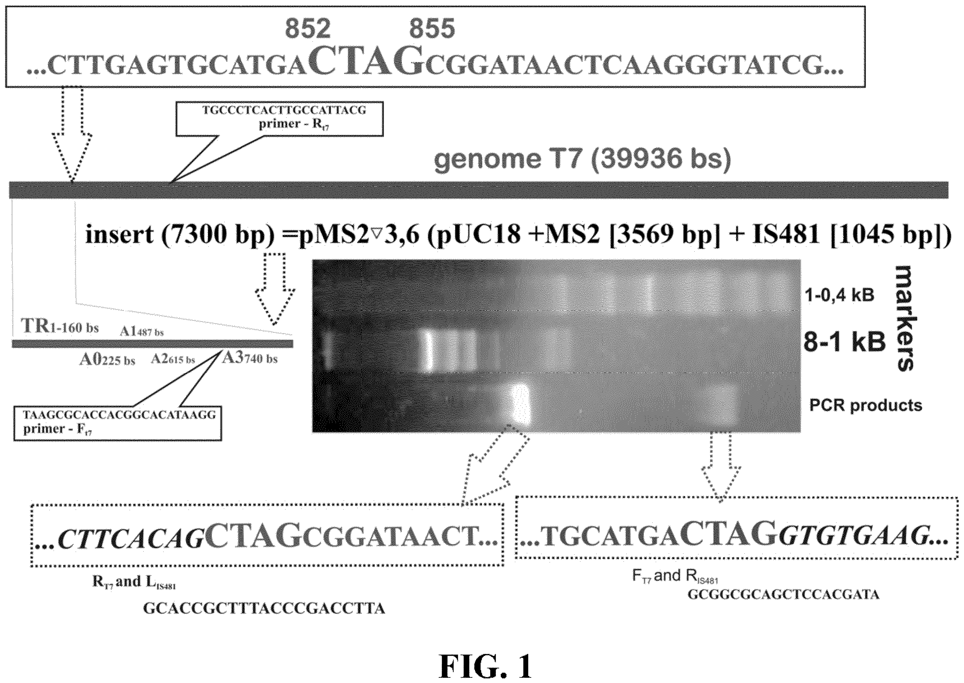

Figure 1 shows the structure of T7 cointegrates::pMS3,6 VRep (or isolates fr; M12; DL1; DL16; J20; ST4; R17 - SEQ ID NO 1) or T7::pGA2,6 VRep (or isolates KU1; T72; DL10; DL20- SEQ ID NO 2).
1 A. The integration site is located in the 0.5kb fragment of BsaBI-BstBI and after A3 promoter, which hinders any transcription towards the ORF of RNA polymerase of T7 phage. The insert is flanked by the direct full-length IS 481 and tetranucleotide CTAG copies. The sequences at the places of "junctions" are displayed in the rectangles shown by a dotted line. The sequences were obtained by analyzing the PCR products obtained from the primers with the structure shown in the figure.
1 B. The PCR amplification of the experimental data. The direction of the electric field is from right to left. The PCR amplification mixture using primer oligonucleotides (* F**_{T7}** - TAAGCGCACCACGGCACATAAGG (SEQ ID NO 3);
   * R**_{IS481}** - GCGGCGCAGCTCCACGATA (SEQ ID NO 4);
   * L**_{IS481}** - GCACCGCTTTACCCGACCTTA (SEQ ID NO 5);
   * R**_{T7}** - TGCTCCTTCCACTGCTTCATCTG) (SEQ ID NO 6).

The primers for PCR were added at the final concentration of 1.5 mM. An upper line represents the PCR products (slow ~ 4B, fast ~ 0<5B); a middle line represents the markers with the fragment size of 10, 9, 7, 5, 3, 1 (B); a lower line represents the markers with the fragment size of 1,000, 900, 800, 700, 600, 500 and 400 (b). The DNA sequence was performed according to the Manual 373 Automatic Automatic Sequencer (Applied Biosystems, USA) using the kit for Dye Doing Terminator ABI sequencing with Taq polymerase FS (Perkin Elmer, USA). The experimental data files (an EMBL format) were compared with the sequences (GenBank sequence acc. No J02518).

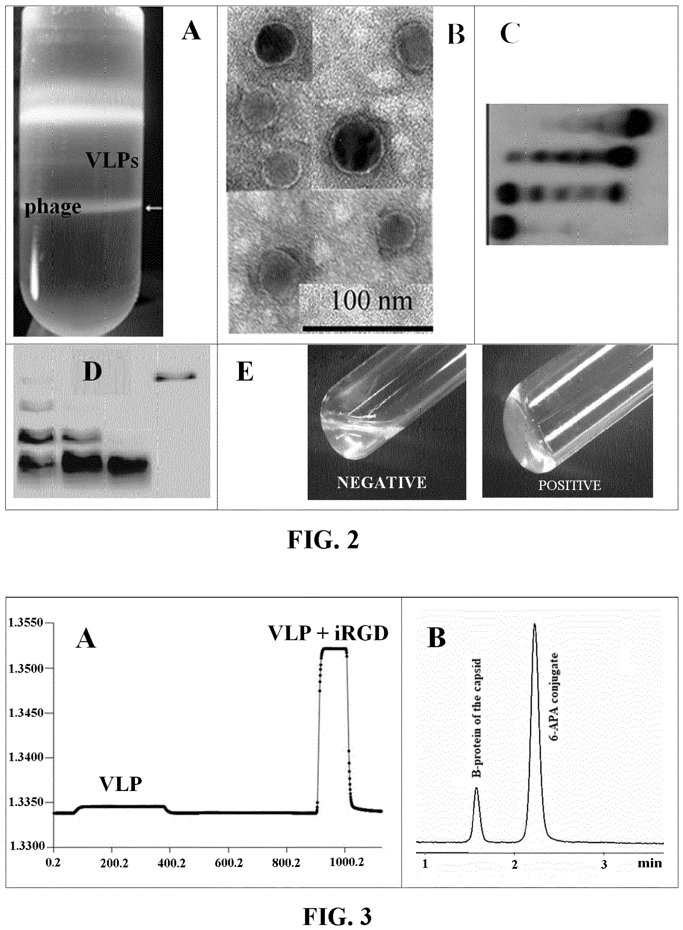

Figure 2 shows obtaining and analysis of the particles of the phages of the genus Lentivirus (the typical results are given).

2A. The gradient centrifugation of the nanoparticles of the phages of the genus Levivirus: the arrows with the designations indicate the accumulations of the particles with a buoyant density known to those skilled in the art. VLPs (the clusters located above, because they are lighter), and the phage particles (the clusters located below, because they are heavier).

2B. Microscopic photographs of the particles of MS2 phage (filled with silver salts; the dark particles up to 30 nm in size, transmission microscopy) or filled with silver salts; the particles up to 25 nm in size, transmission microscopy).

2C. The result of hydrolysis of the genomic RNA in bacteriophages with the changed sequences of the basic protein of the shell. The lower track is the hydrolysis time of 20 minutes, the uppermost one is 4 hours. "Northern dot blot hybridization" (Yadetie F., A.K. Sandvik, H. Bergum, K. Norsett, A. Laegreid. Miniaturized fluorescent RNA dot blot method for rapid quantification of gene expression// BMC Biotechnology 2004, v.4(No 12), pp. 472-480) with a 3-OH labeled oligonucleotide with structure ACATGAGGATTACCCATGT (SEQ ID NO 7) was used to identify the position of the fragments of the phage genomic RNAs.

2D. The overexpression of the genes of the cloned genes of the bacteriophages of the Levivirus family.

PAGE-electrophoresis of the lysates of the warmed up cells (from left to right, every 5 hours from the start of warming up). The lower band is the capsid MS2 protein.

2E. The result of the determination of LPS (a LAL test) in the pseudolysogenic cells. The lysate of the cells of Limulus amebocytis causes a gel formation in the presence of the LPS (> 1pg/ml). The guidance for conducting the test is approved by the FDA USA (Guidance for Industry: Pyrogen and Endotoxins Testing: Questions and Answers, JUNE 2012).

Figure 3 shows the determination of the structure of the obtained VLPs.

3A. By the SPR method (a surface plasmon). The surface of the glass chip treated with anti-poly-lysin IgG1 rabbit antibodies (slight nonspecific binding with the control phage particles - OD = 0.0020 and highly specific binding with anti-poly-lysin IgG1 - OD = 0.230).

3B. By the HPLC method (high-performance high-pressure liquid chromatography). The calculated molecular weight of B-protein of the phages of the genus Levivirus (control - 13866.58) and the obtained VLPs (experiment - 14457.38).

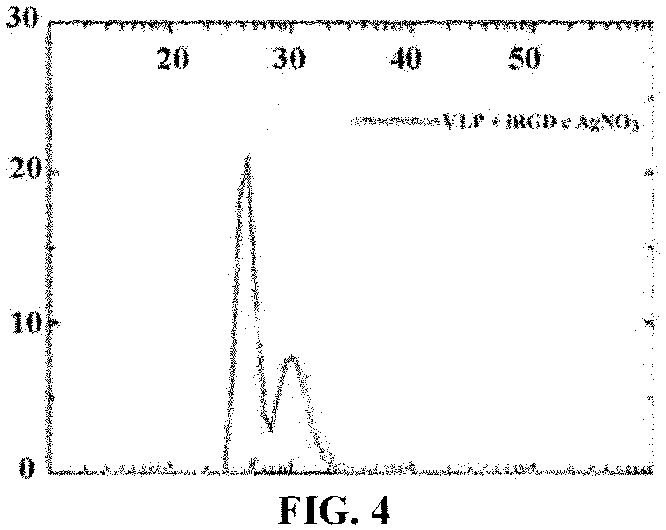

Figure 4 shows the change in the size of the obtained VLPs after filling them with AgNOs (the concentration is 25 mM).

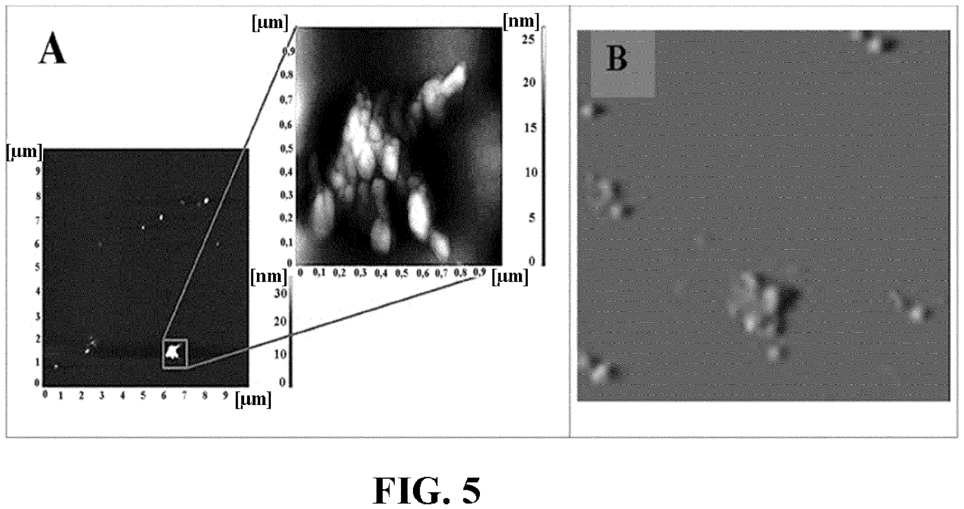

Figure 5 shows AFM (atomic force microscopy) of MS2 bacteriophage (the upper pair of figures) and the obtained phages of the genus Levivirus treated with anti-poly-lysin IgG1 antibodies (the lower pair of figures).

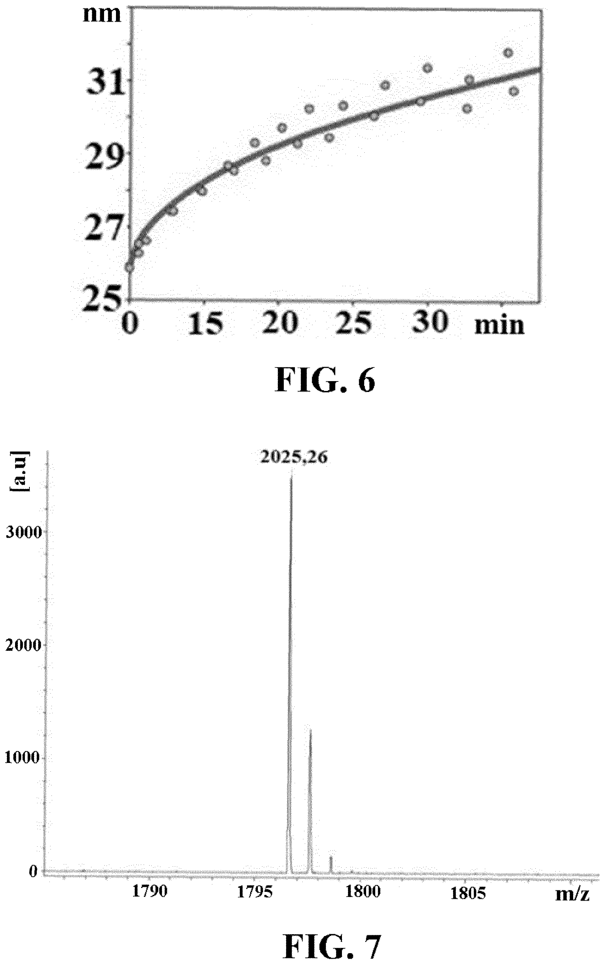

Figure 6 shows the modification of the particles of the phages of the genus Levivirus by the peptide (HHQG)**₄** (SEQ ID NO 8), which causes a change in the size of the particles with a poly-lysin insert.

Figure 7 shows the non-enzymatic conjugation of peptidyl-6APA with B protein of the Levivirus particles.

The offered method for obtaining the phage particles of the genus Levivirus is implemented on the basis of the state of the art by the following sequence of actions.

Initially, the preparatory procedures are performed to obtain high titers and subsequent purification of the samples of MS2 phage or the VLPs phages of the genus Levivirus, as well as a synthesis of the necessary ligands in the form of the organic compounds used to modify the internal surface of the capsids' cavity. For filling with the above compounds, they are used in the form of solutions that are similar to those described, for example, in patents and patent applications (US Patent Applications 20190255088; 20190255087; 20190201547; 20190167584; 20190240186; 20190170742; 20180071399; 20190117561; 20190091165; 20190015526; 20190015320; 20180353613; 20180344849; 20180243440; 20180243229; 20180133343; 20180028683 20180021265; 20180021259; 20170239369; 20150157571) and which, in accordance with this group of inventions, are considered as the core of the modified VLPs phages of the genus Levivirus.

The control of these operations is carried out using the methods of:
- isolating the genomic RNAs;
- calculating the primers for RT-PCR (reverse transcription coupled with PCR);
- reverse transcription and cloning the cDNA;
- electrophoresis of the DNA in agarose;
- transmission electron microscopy (TEM);
- atomic force microscopy (AFM);
- surface plasmon resonance (SPR) (V.N. Konopsky et al. Photonic Crystal Biosensor Based on Optical Surface Waves // Sensors 2013, 13(20), pp. 25662578);
- fluorescence spectroscopy of the samples comprising various metal medications to determine the effectiveness of the packaging and the intensity of washing out (the manipulations were carried out taking into account the letter of the Chief State Sanitary Doctor of the Russian Federation dated 2004.01.06 No 2510/92-04-32);
- isotope analysis to facilitate ion detection, both when assessing the effectiveness of the packaging and to assess the intensity of washing out;
- flame atomic photometry (FAP);
- enzymatic mapping with trypsin (TEC);
- high-performance high-pressure liquid chromatography (HPLC);
- the time of flight mass spectroscopy (MS) and MADI-TOF mass spectroscopy.

The group of inventions is explained by the following examples.

Example 1. Obtaining the particles of MS2 phage and the modified VLPs of the genus Levivirus.

The plasmids were transferred to the cells of Pseudomonas putida using Hft transduction by T7 bacteriophage (Bolshakova T.N., Dobrynina O.N., Karataev G.I., Sivov I.G. Pseudolysogeny in T7 phage and its use // Bacteriophages: theoretical and practical aspects of application in medicine, veterinary medicine and food industry. The Third International Scientific Conference, Moscow, 2016). The control of the infectivity of the particles was carried out according to the method (Knyazhev V.A., Sergienko V.I., Sivov I.G. RNA transduction by non-infectious virions of MS2 phage// Molecular genetics, microbiology and virology. 2002, No 3, pp. 56-63). The resulting electrophoretically pure particles were stored at the temperature of +4° C.

Figure 1 shows the structure of the cointegrates, and Table 1 shows the efficiency of the structure transfer between the representatives of the Enterobacteriaceae family.

**Table 1.**

| T7::pMS3,6 ∇Rep or T7::pGA2,6 ∇Rep transduction of Gram-negative bacteria | | | |
|---|---|---|---|
| **No** | T7::pMS3,6∇Rep | T7::pGA2,6∇Rep | Species |
| | frequency (x 10 ⁶) | frequency (x 10 ⁶) | |
| 1 | 1 - 1.5 | 1 - 1.5 | Escherichia coli |
| 2 | 1 | 1 | Salmonella enterica 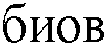 ap Typhimurium |
| 3 | 0.5 | 0.5 | Shigella flexnery |
| 4 | 1 | 1 | Bordetella parapertussis |
| 5 | 0.3 | 0.3 | Pseudomonas aeruginosa |
| 6 | 0.7 | 0.7 | Pseudomonas putida |
| 7 | 0.2 | 0.2 | Erwinia carotovorum |
| 8 | 0.05 | 0.05 | Yersinia enterocolitica |
| 9 | 0.005 | 0.005 | Francisella spp |
| 10 | 0.05 | 0.05 | Brucella abortus |

Example 2. Obtaining the particles of the phage of the genus Levivirus with the fully hydrolyzed RNA.

The bacteriophage particles are treated with diethylamine (DEA 1.5% causes a complete hydrolysis of the genomic RNA during 2 days of incubation at the temperature of 37° C). The control of the decrease in the molecular weight of the genomic RNA was carried out electrophoretically (in 2% agarose gel). The obtained particles were concentrated by centrifugation in a density gradient of CsCl (as it is known to those skilled in the art), as well as the purity of the medications was monitored by transmission electron microscopy according to the method (US Patent 10446366). The particles were stored at the temperature of +4° C under conditions known to those skilled in the art.

Example 3. Obtaining the VLPs of the particles with (HHQG)**₄**.

3A. Performed according to the previously described method [Hooker J.M., E.W. Kovacs, M.B. Francis. Interior Surface Modification of Bacteriophage MS2//J. Supporting Information//J. AM. CHEM. SOC. 2004, v.126 (12), pp. 3718-3719] with the modification of Tyr**₈₅** → Cys in the B-protein sequence (SEQ ID NO 9).

3B. The peptides were obtained by the method of automatic solid-phase synthesis based on 9-fluorenyl(methoxycarbonyl)-amino acids (ChemPep, USA) (synthesizer 433A, Applied Biosystems, FastMoc method). The formation of S-S-bridge was carried out by oxidation of I₂ [10]. The peptide was purified by HPLC at the reversed phase (column C18 Triart, 21 × 250 mm, 10.0 µm, YMC, Switzerland, workstation Agilent 1100, Agilent, USA) in a concentration gradient of CH₃CN (BioSolve, Israel) in water in the presence of 0.1% acetic acid. According to the data of the analytical HPLC at the reversed phase (column C18 Triart, 2.1 × 50 mm, 2.0 µm (YMC), workstation Agilent 1200) with UV and mass spectrometric detection, the purity of the peptide medication is not lower than 95%.

3C. The non-enzymatic reaction of the addition of iRGD-6APA (SEQ ID NO 10)

The conjugation of the peptide with capsid B-protein of the VLPs of the genus Levivirus was performed using the homobifunctional reagent dimethyladipimidate (DMAI, Sigma, USA) in the molar ratio the phage protein: the peptide: DMAI 1:20:80 according to the method [US Patents 6080383; 6468503; 10576144 and DE Patent 000069731747] in 40% dimethylsulfoxide (DMSO). The VLPs were separated from the excess of the reagents by precipitation with a 25% solution of polyethyleneglycol 6000 ( 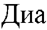 -M, Russia) using 1M NaCl. The precipitated VLPs were suspended in deionized water.

Example 4. Biomineralization of the particles.

At the preliminary step, the desired shRNA structure (for binding with the maximum number of cations; at least 200) was calculated using the program https://biosettia. com/support/shrna-designer/ and synthesized according to the method [Chris B. Moore, E. H. Guthrie, M. Tze-Han Huang, D. J. Taxman. Short Hairpin RNA (shRNA): Design, Delivery, and Assessment of Gene Knockdown//Methods Mol Biol. 2010 ; 629: 141-158]. The administration of the shRNA in a complex with the salts was carried out with TMAO [RF Patent 2599462] and taking into account the calculations obtained earlier [Farafonov V.S., Nerukh D. MS2 bacteriophage capsid studied using all-atom molecular dynamics//Interface Focus. 2019 Jun 6;9(3): pp. 81 - 85]. The "loosened" particles were transferred from pH = 4.5 to the sodium carbonate buffer (0.5 M, pH = 10.0). The salts and metal medications were transferred to the solution at the concentration of at least 1/50 stock solutions (< 1 mg/ml) in the ratio of 100 µg of the protein. Then incubated during 30 min, with gentle stirring on a magnetic stirrer. Then the mixture was dialized in a pre-cooled glass tube on ice, left on ice for 10 minutes, and then for 5 minutes at the room temperature. The residues of the salts and metal medications were removed from the samples by gel filtration using Sephadex G-50. The medications were added to the columns with Sefadex. The separation was repeated twice. The final concentrations of the salts encapsulated in nanoparticles were evaluated using a flame photometer (PFP-7/C, Jenway, England). The results were compared with the curve of the concentrations of the compounds in the wavelength range of 280-800 nm. The size of the loaded particles was measured using a particle size analyzer (IG-1000 Plus, Shimadzu, Japan).

The analysis was carried out according to the manual instructions for the analytical method of flame photometry [Flame Atomic Absorption Spectrometry. Analytical Methods / /Fourteenth edition, 2017 Agilent Technologies Australia (M) Pty, Ltd.]. The binding of the metal cations to B-protein was monitored using the program "Metal Detector v.2.0". The control measurements of the concentration of the substances gave a linear dependence with r > 0.98. The results obtained are summarized in a table known to those skilled in the art [Analytical Methods for Atomic Absorption Spectroscopy. Manual Perkin Elmer. 1996].

Example 5. Atomic-powered microscopy of the phage particles of the genus Levivirus.

Carried out according to the scheme described earlier [Kuznetsov Y.G., Sheng-Chieh Chang, A. McPherson. Investigation of bacteriophage T4 by atomic force microscopy//Bacteriophage. 2011 May-Jun; 1(3): 165-173; A. Ramanaviciene, V. Snitka, R. Mieliauskiene, R. Kazlauskas, A. Ramanavicius. AFM study of complement system assembly initiated by antigen-antibody complex//Central European Journal of Chemistry 2006, v.4(1), pp 194-206]. The results are summarized in Figures 5A and 5B.

5a. The chip preparation.

Piece of mica (Structure Probe, Inc., West Chester, PA, USA) was used for the manufacture of the chips, which was sialized in pairs of 3-aminopropyltriethoxysilane (APTES), as known to those skilled in the art. The surface of the sialized mica substrates was activated with 0.12 mM DTSSP solution in 10 mM PBSD (pH 7.4) during 10 min, washed with 1 ml of 50% absolute ethanol during 10 min at the temperature of 10° C and then dried in an nitrogen stream, immediately used for immobilization. The incubation solutions (~ 1 mcl) were carefully dripped onto the surface of the activated substrate, incubated during half an hour at the room temperature (25° C) in a moist chamber.

5_{B}. AFM scanning was performed using a titanium atomic-powered microscope (NT-MDT, Zelenograd, Moscow, Russia). NT-MDT has the resonant frequency of 47-150 kHz, the radius of the curvature of the tip of the cantilever is 10 nm). The AFM data were assessed according to two criteria: the first is "qualitative," which was based on an obvious increase in the height of the complexes compared to the height of the irregularities of the chip. Triangular sensors with wide legs of 13 µm and the total length of 100 µm were used. The claimed constant of the force (0.21 N/m) was checked with an accuracy of up to 20% by measuring the deflection of the cantilever when pressed against a quartz fiber of the known stiffness. The scanning head was calibrated in the x,y and z directions using polystyrene-latex stands, as known to those skilled in the art. There is some uncontrolled change from launch to launch, which may be related to the scanners, and this introduces a systematic error in sizes up to ± 2.5%. The experiments were carried out in a liquid cell, using air and deionized water. The height and width of the image were measured using software available on workstation NanoScope Im. At least 40 molecules were measured at 5 points along their contours for each material shown here. The results were analyzed by program KaleidaGraph.

The force measurements were made using a force display on the AFM (A graph of the dependence of the voltage applied to the control of the height of the sample associated with the position of the sample, Zs, through the known height calibration, from the deviation signal associated with the force through the deflection of the tip, ZT and the constant of the cantilever spring, kH). The adhesion can vary significantly, as it depends on the uncontrolled geometry of the tip and on contamination. We present the averaged values and the range that reflects the typical conditions of the experiment (see Figures 5A and 5B). Table 2 shows the measurement results using AFM.

**Table 2.**

| The VLPs of the genus Levivirus | | | |
|---|---|---|---|
| | Air | Water | 40% alcohol |
| Length (nm) | 25.5±2.3 | 22.6±4.5 | 23±7 |
| Width (nm) | 27±1.3 | 26±3 | 26±5.6 |

| The VLPs of the genus Levivirus with filling | | | |
|---|---|---|---|
| | Air | Water | 40% alcohol |
| Length (nm) | 122.5±22.3 | 122.6±28.5 | 103±72 |
| Width (nm) | 127±21.3 | 156±32.6 | 126±35.3 |

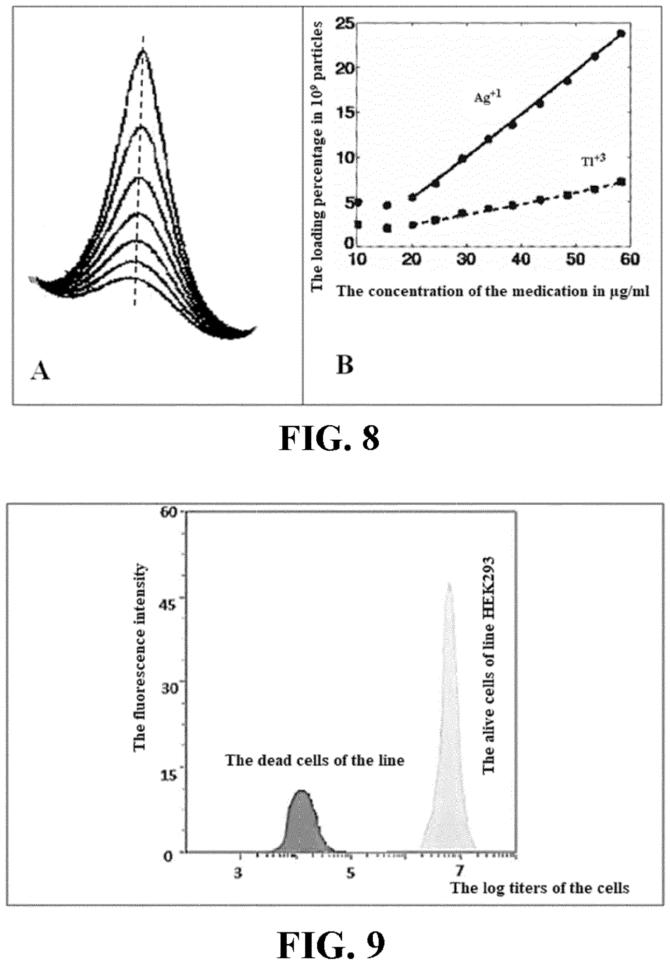

Example 6. The isotope analysis of the detection of a stable isotope (see Figures 8A and 8B).

The isotope analysis of the detection of an ion was carried out according to (Good Practice Guide for isotope ratio mass spectrometry 2011, first edition; as well as Park S.K., Venable J.D., Tao Xu, John R. Yates III. A quantitative analysis software tool for mass spectrometry- based proteomics//Nat Methods. 2008; v.5(No.4), pp. 319-322 and Ilbeigi V., Y. Valadbeigi, M. Tabrizchi. Ion Mobility Spectrometry of Heavy Metals//Anal. Chem. 2016, v.88 (No 14), pp. 7324-7328).

Example 7. The toxicity (mg/kg) of the medications and metal salts was determined for mice (DCL; according to ISO/IEC 17025:2017 [General requirements for the competence of testing and calibration laboratories] and GOST ISO/IEC 17025- 2009), as well as for cell cultures (d**_{aggl}**). The toxicity of the medications and the metal salts for mice is shown in Table 3.

**Table 3.**

| Medication | DCL (µg/dl) | in 10**⁸** particles (ng/dl) | Therapeutic ratio × 10⁻⁷ | d**_{aggl}** (pg/dl) | dCL/ d**_{aggl}** |
|---|---|---|---|---|---|
| 1 | 2 | 3 | | 4 | 5 |
| cisplatin (*cis*-Pt(N H**₃**)**₂** Cl**₂**) | 3.5 | 0.5 | 7 | 2 | 5 |
| (NH**₄**)**₂**[PtCl**₆**] | 0.1 | 0.02 | 5 | 0.5 | 2 |
| Ru(NO**₃**)**₃** | 2.0 | 0.03 | 67 | 1 | 1 |
| AuH**₂**Na**₃**O**₆**S**₄** | 0.5 | 0.01 | 50 | 10 | 5 |
| Fe(C**₁₂**H**₂₂**O**₁₁**)**₃** | 25 | 0.01 | 250 | 1 | 5 |
| AgNO**₃** | 9 | 0.05 | 180 | 2 | 1 |
| Cu(NO₃)₂ | 15 | 0.6 | 25 | 3 | 1 |
| Zn(NO**₃**)**₂** | 5 | 0.1 | 50 | 1 | 0.5 |
| Ga(NO**₃**)**₃** | 35 | 3.8 | 9.2 | 20 | 8 |
| NaVO**₃** | 2.0 | 0.03 | 67 | 1 | 1 |
| Tl**₂**(CO**₃)₃** | 19 | 1.3 | 14.6 | 9 | 0.5 |
| Tl**₂**CO**₃** | 21 | 1.6 | 13.1 | 12 | 2 |

Example 8. The targeted delivery of the particles of the phages of the genus Levivirus.

The accuracy of the delivery was qualitatively identified by the method of flow fluorimetry from the mixture of the cells enriched in integrin α**ᵥ**β**₃** and devoid of it (lines PC3 and HEK293). For this purpose, the mixtures of the cells with and without integrin were prepared in various ratios (1:10; 1:20 and 1:40) in the titer of 10⁷/ml. The VLPs of the genus Levivirus were added to the cells with filling, and after 3 hours a fluorescent dye 4',6-diamiidno-2-phenylindole (DAPI) was added to stain the dead cells. The results obtained are summarized in Figure 9 and in Table 4. The calculated correlation coefficients for the lethal effect of the particles of VLPs of the genus Levivirus with filling are also shown in Table 4.

**Table 4.**

| The medication for filling and the correlation coefficient obtained in the experiments with the PC3 line in the mixture with iRGD + 6APA ligand | The ratio of the log titers of PC3/HEK293 cells | % of HEK293 death in the mixture with iRGD + 6APA ligand | % of PC3 death in the mixture with iRGD + 6APA ligand | % of HEK293 death in the mixture with 6APA ligand | % of PC3 death in the mixture with 6APA ligand |
|---|---|---|---|---|---|
| cisplatin (*cis*-Pt(N H**₃**)**₂**Cl**₂**) | 1 | | 95 | | |
| | 2 | | 88 | | |
| R= 0.90 | 3 | | 82 | | |
| (NH**₄**)**₂**[PtCl**₆**] | 1 | | 95 | | |
| | 2 | | 92 | | |
| R= 0.92 | 3 | | 90 | | |
| Ru(NO**₃**)**₃** | 1 | | 85 | | |
| | 2 | | 70 | | |
| R= 0.75 | 3 | | 55 | | |
| AuH**₂**Na**₃**O**₆**S**₄** | 1 | | 75 | | |
| | 2 | | 70 | | |
| R= 0.72 | 3 | | 56 | | |
| Fe(C**₁₂**H**₂₂**O**₁₁**)**₃** | 1 | | 60 | | |
| | 2 | | 45 | | |
| R= 0.65 | 3 | | 22 | | |
| AgNO**₃** | 1 | | 80 | | |
| | 2 | | 75 | | |
| R= 0.82 | 3 | | 65 | | |
| Cu(NO**₃**)**₂** | 1 | | 85 | | |
| | 2 | | 82 | | |
| R= 0.85 | 3 | | 65 | | |
| Zn(NO**₃**)**₂** | 1 | | 50 | | |
| | 2 | | 35 | | |
| R = 0.6 | 3 | | 20 | | |
| Ga(NO**₃**)**₃** | 1 | | 90 | | |
| | 2 | | 82 | | |
| R= 0.75 | 3 | | 75 | | |
| NaVO**₃** | 1 | | 70 | | |
| | 2 | | 55 | | |
| R = 0.65 | 3 | | 40 | | |
| Tl**₂**(CO**₃**)**₃** | 1 | | 95 | | |
| | 2 | | 92 | | |
| R= 0.92 | 3 | | 89 | | |
| Tl**₂**CO**₃** | 1 | | 95 | | |
| | 2 | | 85 | | |
| R= 0.90 | 3 | | 80 | | |

## Claims

1. A method for obtaining the particles of the bacteriophages of the Levivirus family, including the following steps of:
(a) obtaining a line of pseudolysogenic the host cells multiplying at a temperature of ≤ 23°C, devoid of toxic lipopolysaccharide and containing:
- a sequence of the nucleic acid encoding T7 phasmid with a fragment of the genomes of bacteriophages of the Levivirus family;
- a sequence of the nucleic acid of T7 phasmid encoding the proteins of the capsid of the particles of the bacteriophages of the Levivirus family;
- a sequence of the nucleic acid of T7 phasmid encoding the proteins of the DNA- and RNA-polymerase of T7 phage and devoid of the morphogenesis genes;
(b) initiating overexpression of the genes encoding the proteins of the capsid of the particles of the bacteriophages of the Levivirus family at an increase in temperature ≥ 28° C;
(c) obtaining the particles of the bacteriophages of the Levivirus family with simultaneous destruction of the host cells, wherein these bacteriophage particles have morphological similarity with the natural phage particles.

2. The particle of the bacteriophage of the Levivirus family obtained by the method according to claim 1, devoid of LPS toxicity.

3. The method of biomineralization of the particles of the bacteriophages of the Levivirus family according to claim 2, obtained by the method according to claim 1, designed to reduce significantly the overall toxicity of the particles loaded with metal medications, including the steps of:
(a) complete removal of RNA from the particles of the bacteriophages of the Levivirus family;
(b) obtaining a complex of the metal medications with hairpin RNA (shRNA) binding at least 200 cations;
(c) "loading" the complex of the metal medications with hairpin RNA (shRNA) binding at least 200 cations, into the particles of the bacteriophages of the Levivirus family, devoid of RNA, "loosened" by trimethylamine N-oxide;
(d) a non-enzymatic attachment of a ligand to the biomineralized particles suitable for creating a targeted delivery system to malignant tumor cells, including to the cells enriched in ανβ3 integrin;
(e) determination of the delivery specificity and sensitivity using a mixture model of a PC3 cell line, one of which is enriched enriched in ανβ3 integrin.

## Amended claims

### Amended claims under Art. 19.1 PCT

1. A method for obtaining the particles of the bacteriophages of the Levivirus family, including the following steps of:
(a) obtaining a line of pseudolysogenic the host cells multiplying at a temperature of ≤ 23°C, devoid of toxic lipopolysaccharide and containing:
- a sequence of the nucleic acid encoding T7 phasmid with a fragment of the genomes of bacteriophages of the Levivirus family;
- a sequence of the nucleic acid of T7 phasmid encoding the proteins of the capsid of the particles of the bacteriophages of the Levivirus family;
- a sequence of the nucleic acid of T7 phasmid encoding the proteins of the DNA- and RNA-polymerase of T7 phage and devoid of the morphogenesis genes;
(b) initiating overexpression of the genes encoding the proteins of the capsid of the particles of the bacteriophages of the Levivirus family at an increase in temperature ≥ 28° C;
(c) obtaining the particles of the bacteriophages of the Levivirus family with simultaneous destruction of the host cells, wherein these bacteriophage particles have morphological similarity with the natural phage particles.

2. The method of biomineralization of the particles of the bacteriophages of the Levivirus family, obtained by the method according to claim 1, designed to reduce significantly the overall toxicity of the particles loaded with metal medications, including the steps of:
(a) complete removal of RNA from the particles of the bacteriophages of the Levivirus family;
(b) obtaining a complex of the metal medications with hairpin RNA (shRNA) binding at least 200 cations;
(c) "loading" the complex of the metal medications with hairpin RNA (shRNA) binding at least 200 cations, into the particles of the bacteriophages of the Levivirus family, devoid of RNA, "loosened" by trimethylamine N-oxide;
(d) a non-enzymatic attachment of a ligand to the biomineralized particles suitable for creating a targeted delivery system to malignant tumor cells, including to the cells enriched in ανβ3 integrin;
(e) determination of the delivery specificity and sensitivity using a mixture model of a PC3 cell line, one of which is enriched in ανβ3 integrin.

Statement under Art. 19.1 PCT
Dear ladies and gentlemen,
 In response to the Notification on sending the international search report and the written communication of the international search authority or declaration dated 10.12.2020, as well as in accordance with Article 19 of the Patent Cooperation Treaty, we send you the amended claims of international application PCT/RU2020/000198.

The Applicant, having read in detail the international search report, agrees with the arguments of the examination that in the cited reference D1: PEABODY J.ET AL. Characterization of a spray-dried HPV L2-VLP vaccine stored for multiple years at room temperature. Papillomavirus Research 3 (2017) 116-120 (see sections 2.1., 2.2) obtaining MS2 bacteriophage particles (the Levivirus family) devoid of LPS toxicity is disclosed. Thus, the invention according to claim 2 is not new. At the same time, according to independent claims 1 and 3, the invention is recognized as corresponding to the conditions of patentability "novelty", "inventive step" and "industrial applicability,"

In this regard, the Applicant, in the submitted amended claims according to Article 19 of the Patent Cooperation Treaty, excludes independent claim 2, wherein leaving remaining claims 1 and 3 unchanged, he corrects only the numbering of the sequence of claims and removes the reference to excluded claim 2.

Thus, the amendments made to the claims do not change the application in essence.

The Applicant hopes that he has fully taken into account the expert's comments given in the Written Communication of the international search authority, and requests to accept these amendments in the international application.

Appendix:
 1. The amended claims of application PCT/RU2020/000198 according to Article 19 of the Patent Cooperation Treaty on 1 page in 3 copies.
